# EUROPEAN PATENT SPECIFICATION

(11) **EP 2 634 247 B1**
(45) Date of publication and mention of the grant of the patent: **10.09.2014**
(21) Application number: 12382074.8
(22) Date of filing: 29.02.2012
(51) Int. Cl.: C12N 1/18, C12G 1/022, C12R 1/865

(54) **Strain of saccharomyces cerevisiae and its use for the production of alcoholic beverages**
Saccharomyces cerevisiae Stamm und dessen Verwendung bei der Herstellung alkoholischer Getränke
Souche de Saccharomyces cerevisiae et son utilisation dans la production de boissons alcoolisées

(43) Date of publication of application: 04.09.2013
(73) Proprietor: Guserbiot S.L.U., 01015 Vitoria Alava (ES)
(72) Inventor: Abadin Insua, Cristina, 01015 Vitoria (ES); Barbero Mangas, Francisca, 01015 Vitoria (ES); Oyanguren García, Iñigo, 01015 Vitoria (ES); Zumárraga Uribesalgo, Miren, 01015 Vitoria (ES)
(74) Representative: Pons Ariño, Angel

(56) References cited:
- EP-A1- 1 482 029
- EP-A1- 2 277 990
- WO-A1-2011/004254

## Description

The present invention belongs to the field of the production of yeasts that are useful in the elaboration of alcoholic beverages by fermentation processes; specifically, the invention relates to a new *Saccharomyces cerevisiae* strain and to the use thereof for that purpose.

### PRIOR STATE OF THE ART

Until very recently, particularly in Old World oenology, the predominant idea was that the spontaneous fermentation of grape must, i.e. that which takes place without using any external inocula, resulted in wines with better aromas and bouquets. Currently, however, it is assumed that the use of selected yeasts presents numerous advantages with respect to traditional spontaneous fermentation, such as a higher rate of fermentation, a maximum consumption of reducing sugars, a greater reproducibility of the quality of the wine elaborated, the prevention of chemical and microbiological alterations during the early fermentation stages and a greater flexibility in controlling the sensory quality of the wine.

In fact, the selected yeasts have been used with excellent results in many countries, to obtain final products with a more uniform quality than those produced by means of spontaneous fermentations.

Wine is the product obtained from alcoholic fermentation, a process whereby grape sugars are converted into ethanol due to the action of yeasts. However, the true significance of the latter lies not only in the production of the alcoholic degree of the wine obtained, but, furthermore, in the fact that, during the fermentation process, the yeasts are involved in the generation of numerous secondary compounds that determine the final quality of the wine elaborated, as well as the typicality thereof.

During fermentation of the grape must, the sequential action of different genera and species of yeasts takes place. During the first fermentation stages (4 % - 5 % vol. of alcohol), indigenous yeasts with a low fermentation power are developed, of the following genera: *Kloeckera, Hanseniaspora, Candida and Pichia.* Once a 4 % - 5 % vol. of alcohol is exceeded, the fermentation process is primarily dominated by the species *Saccharomyces cerevisiae,* which is more resistant to alcohol (Heard and Fleet, 1988, Journal of Applied Bacteriology, 65: 23-28).

The origin of these yeasts lies primarily on the surface of grapes, as well as the cellar environment (machinery, hoppers, presses, fermentation reservoirs, etc.), the latter being a significant source of microorganisms that inoculate the grape musts. The number of yeasts present in the skin of healthy grapes is very low (between 10³ and 10⁶ CFU/ml), and the number of *Saccharomyces cerevisiae* is even lower (less than 50 cells/ml of must). In cellars, *S*. *cerevisiae* is the predominant species (Fleet, 2003, International Journal of Food Microbiology, 86: 11-22).

The microflora present in grapes may be affected by a large number of factors which have an influence on the number and proportion of the different species. These factors include the temperature, the pluviometry and other climactic factors, the degree of maturity of the grapes, the use of phytosanitary treatments, the physical damage caused by fungi, insects, etc., and the grape variety. Changes in the initial flora may affect the fermentation process, causing delays in fermentation and even stucks, may influence the wine quality, and lead to changes in the volatile acidity and to unpleasant flavours and/or aromas.

In fact, the variability of the yeast-like flora of grape musts may be resolved by the addition, in each grape harvest, of a microbial inoculum which, since it is predominant, normalises the initial flora and, thus, leads to homogeneous fermentation in each cellar year after year.

When selecting the yeasts for the production of quality wines, it is desirable that they have the following characteristics:
- High tolerance to ethanol.
- Total degradation of the fermentable sugars.
- Resistance to SO_{2.}
- High fermentation capacity.
- Production of glycerol.
- *Killer* phenotype.
- Fructophilic character.
- Resistance to fermentation stress.

On the contrary, it is intended to prevent the yeast from having the following undesirable characteristics:
- Production of H₂S.
- Production of volatile acidity.
- Production of acetaldehyde and pyruvate.
- Formation of ethyl carbamate precursors.

Currently, almost all cellars use commercial yeast inocula for the fermentation of their grape musts. The use of these commercial inocula in the cellars reduces the risk of slow fermentation kinetics and defects in the taste and the aroma of the wines. For this reason, the inoculation of grape must with these yeasts is a widespread practise in cellars. However, in most cases these yeasts have been isolated in areas, from grape varieties and under conditions different from those in the cellars that use them, in addition to being used interchangeably in other designations of origin, both Spanish and from other countries, the consequence of this use being the elaboration of wines with a certain loss of typicality.

One alternative that is turning out to be valid to overcome the disadvantages of generalised application and the loss of typicality is the use of autochthonous yeasts, isolated from grapes in a given area, to elaborate the wines from this same area. In this way, the fermentation process may be controlled whilst at the same time respecting the local character of the wines.

Such autochthonous yeasts isolated from different wine-growing districts are disclosed in EP 1 482 029, EP 2 277 990 and WO 2011/004254.

EP 1 482 029 discloses *Saccharomyces cerevisiae* strains isolated from musts of D.O. Wines of Madrid having enological properties, such as high fermentative power, high SO₂ resistance and the killer phenotype.

EP 2 277 990 discloses a *Saccharomyces cerevisiae* strain with enological characteristics, which has been isolated from grapes of the Tempranillo variety collected in vineyards in the Rioja wine region.

Another a *Saccharomyces cerevisiae* strain useful in wine making is disclosed in WO 2011/004254. The strain has been isolated from Prosecco grapes cultivated in the Treviso province in Italy.

At this point we must note the numerous advantages for the elaboration of wines offered by the use of autochthonous yeasts selected in the designations of origin. On the other hand, the selection must focus on the grapes, not the cellar environment, given that precisely in cellars there is a residual population of yeasts of a varied, primarily commercial, origin.

### DESCRIPTION OF THE INVENTION

The present invention provides a new strain of the species *Saccharomyces cerevisiae* that is useful for the elaboration of alcoholic beverages by fermentation. As will be seen in the examples, this strain has the advantages of being more resistant to fermentation stress, improving the organoleptic perception of the wine produced, providing a wine with a lower sugar content (more fructophilic character of the strain), a higher glycerol content and a lower volatile acidity, as compared to other commercial strains, both when it is used at the micro-vinification level and when it is used in the cellar. Moreover, said strain presents a better adaptation to low temperatures and initiates the fermentation process earlier than yeasts of commercial origin. It has faster, more regular fermentation kinetics in the presence of high concentrations of sugars, a high tolerance to ethanol and sulfur dioxide, and provides a low production of hydrogen sulfide. Therefore, the strain disclosed herein presents a high, regular fermentation capacity at different fermentation temperatures and sugar contents of the fermented grape must. Moreover, the autochthonous yeast GBiot EL-261, or "strain of the invention", presents a higher mannoprotein content in the structure of its cell wall than other yeasts of commercial origin, which favours protein stabilisation and improves the organoleptic properties of the wine produced.

For all these reasons, a first aspect of the invention relates to a strain of the species *Saccharomyces cerevisiae* deposited in the Spanish Type Culture Collection (CECT) with deposit number CECT 13065. Hereinafter, we will also refer to this strain as "strain of the invention" or "strain GBiot-EL 261". Said strain was deposited on 17 January 2012 at the CECT, Edificio 3 CUE, Parc Cientific Universitat de Valencia, Catedrático Agustin Escardino, 9, 46980, Paterna (Valencia, Spain).

The strain of the invention belongs to the kingdom Fungi, division *Ascomycota,* class *Hemiascomycetes,* order *Saccharomycetales,* family *Saccharomycetaceae,* genus *Saccharomyces*, species *S. cerevisiae.*

In a preferred embodiment, the strain of the invention is found in liquid format, fresh in paste, active dry or instant dry.

The "liquid format" refers to a suspension of yeast cells in a liquid medium, with a solids content of, preferably, 20 % - 25 %.

The "fresh in paste format" refers to a format wherein the yeast is concentrated in a paste, with a dry matter content of, preferably, 30 % - 35 %. It is produced by the filtration of yeast in liquid format.

The "active dry format" refers to a format wherein the yeast is in dry form and has a maximum moisture content of preferably 8 %. It is produced by the fluid-bed drying of yeast in fresh in paste format.

The "instant dry format" refers to a format wherein the yeast is in dry form and has a maximum moisture content of preferably 5 %. It is produced by the fluid-bed drying of yeast in fresh in paste format.

The strain of the invention may be used in a fermentation process, by itself or combined with other microorganisms capable of performing an alcoholic fermentation, preferably with other yeast strains of the same genus or a genus different from that of the strain of the invention, with the associated advantages described above, in order to obtain alcoholic beverages. For this reason, another aspect of the invention relates to the use of the strain of the invention for the elaboration of alcoholic beverages obtained by alcoholic fermentation.

"Alcoholic fermentation" is understood to mean the anaerobic process which, in addition to generating ethanol, releases large quantities of carbon dioxide (CO₂) and energy for the metabolism of anaerobic bacteria and yeasts. Alcoholic fermentation is a biological fermentation process in the absence of air (oxygen - O₂), caused by the activity of some microorganisms that process carbohydrates (as a general rule, sugars, such as, for example, glucose, fructose, sucrose, etc.) to obtain, as the final products, an alcohol in the form of ethanol (the chemical formula whereof is: CH₃-CH₂-OH), carbon dioxide (CO₂) in the form of a gas and ATP molecules which are consumed by the microorganisms themselves in their anaerobic cell energy metabolism.

"Alcoholic beverages" are all those beverages that contain ethanol and include both beverages produced by alcoholic fermentation, such as, for example, without being limited thereto, wine, beer, hydromel, sake, cava or cider, and beverages produced by distillation, generally starting from a fermentation product (liqueurs, eaux-de-vie, etc.), which include beverages with very varied characteristics, from different types of brandy and liqueur, to whisky, anisette, tequila, rum, vodka, cachaça, vermouth or gin, amongst others. In a preferred embodiment, the alcoholic beverage is selected from the list consisting of: wine, beer, cava and cider. In a more preferred embodiment, the alcoholic beverage is wine elaborated from grape musts.

"Wine" is the beverage obtained from grapes by the alcoholic fermentation of the must or juice thereof. The fermentation is produced by the metabolic action of yeasts which convert the sugars in the fruit into ethyl alcohol and gas in the form of carbon dioxide. The sugar and the acids in the grape are sufficient for the development of fermentation. However, wine is the result of a set of environmental factors, such as, for example, without being limited thereto, the climate, latitude, altitude, hours of daylight, etc. The wine whereto the present invention refers may be, without being limited thereto, burning wine (that which is used for distillation since it lacks adequate conditions for human consumption), mouth wine (designed for human consumption, for example as a beverage), sparkling wine, semi-sparkling wine, white wine, red wine, *aloque* (that which results from mixing white and red wine), rosé, saturated wine (dark red, hardly transparent wine), claret (transparent, uncloudy, bright wine) or noble wine (that which does not require the prior addition of sugar to the grape must in order to be elaborated).

The "grape musts" whereto the present invention refers may come from any grape variety known to persons skilled in the art which may be used for the elaboration of wines. In a preferred embodiment, said musts come from grapes from the La Rioja viticultural region, more preferably, they are musts from red grape varieties, even more preferably from the Garnacha, Graciano, Mencia, Monastrell, Mazuelo, Bobal or Tempranillo varieties. In an even more preferred embodiment, the grape is of the Tempranillo variety, even more preferably from the viticultural region of the Autonomous Community of La Rioja. The "Tempranillo variety", also called *"Tinta del país"* or "Cencibel", refers to both the red variety and the white variety of recent mutation: "white tempranillo". Tempranillo is preferably a red grape with a thick skin, which grows better at relatively high altitudes, but may also tolerate much warmer climates.

Another aspect of the invention relates to the use of the strain of the invention for the production of biomass. The term "biomass" refers to the quantity of live yeast cells obtained by means of a fermentation process designed to produce the yeast. Depending on the conditions established to produce the yeast (nutrients, temperature, aeration, pH), the yield of the biomass produced and the physiological state thereof may vary, which has an impact on the fermentation activity of the yeast. Likewise, the drying process is essential, since it may affect the viability of the cells. Therefore, the correct design and development of the production process are essential for the yeast to correctly perform the alcoholic fermentation.

Throughout the description and the claims, the word "comprises" and the variants thereof are not intended to exclude other technical characteristics, additives, components or steps. For persons skilled in the art, other objects, advantages and characteristics of the invention will arise partly from the description and partly from the practise of the invention. The following examples and drawings are provided for illustrative purposes, and are not intended to limit the scope of the present invention.

### DESCRIPTION OF THE FIGURES

Figure 1.- Shows the genetic profiles of strain GBiot-EL 261 obtained by electrophoretic separation in agarose gels using amplification of the inter-delta sequences (A), and the analysis of restriction fragments of mitochondrial DNA (B). M: molecular weight marker (λ phage, digested with the Pstl restriction enzyme).
Figure 2.- Shows the fermentation kinetics of the autochthonous yeast GBiot-EL 261 and the commercial yeast in micro-vinification I. The fermentation kinetics of these yeasts was expressed as the evolution of the °Brix of the grape must as a function of time.
Figure 3.- Shows the fermentation kinetics of the autochthonous yeast GBiot-EL 261 and the commercial yeast in micro-vinification II.
Figure 4.- Shows the fermentation kinetics of the autochthonous yeast GBiot-EL 261 and the commercial yeast in micro-vinification III.
Figure 5.- Shows the fermentation kinetics of the autochthonous yeast GBiot-EL 261 and the commercial yeast in micro-vinification IV.
Figure 6.- Shows the final concentrations of glucose (A), fructose (B), glycerol (C) and acetic acid (D) in the wines inoculated with the commercial yeast and the autochthonous yeast starting from synthetic grape musts under different initial conditions: standard (100 g/I glucose + 100 g/I fructose); excess fructose (50 g/I glucose + 150 g/I fructose); only fructose (200 g/I fructose) and only glucose (200 g/I glucose).
Figure 7.- Shows the study of the resistance of the autochthonous and the commercial yeast following the incubation thereof in different concentrations of the K9 toxin for 5 hours. Growth in YPD plates after 24 hours (A) and 48 hours (B) of incubation. Controls were performed of both the YPD medium and the toxin.

### EXAMPLES

Below we will illustrate the invention by means of assays performed by the inventors, which show the effectiveness of the strain of the invention in fermentation processes for the elaboration of alcoholic beverages. These specific examples are provided to illustrate the nature of the present invention and are included for illustrative purposes only; therefore, they should not be interpreted as limitations to the invention claimed herein. Therefore, the examples described further below illustrate the invention without limiting the field of application thereof.

### EXAMPLE 1. CHARACTERISATION OF STRAIN GBiot-EL 261

### 1.1. Morphology

- Cell morphology: oval, round cells.
- In a solid medium (malt extract agar), the colony presented a whitish colour and a smooth, bright surface.
- In lysine agar, it did not present any growth.
- In a sporulation medium (Kleyn's acetate agar), it formed ascospores, which contained 4 spores per ascus. The spores presented a round morphology.

### 1.2. Metabolic characteristics

- Fermentation of carbohydrates under aerobiosis conditions: the strain was glucose (+), maltose (+), sucrose (+), trehalose (+), raffinose (+), α-glucosidose (+), xylose (+), galactose (+), histidine (+), leucyl-glycine (+), proline (-) and lactose (-).
- Fermentation of carbohydrates under anaerobiosis conditions: the strain was glucose (+), maltose (+), sucrose (+), trehalose (+), raffinose (+), β-glucosidose (+), α-glucosidose (+) and galactose (+).
- Assimilation of nitrogenated compounds: the strain was nitrate (-), lysine (-).
- Oxygen requirements: the strain is facultative anaerobic.
- Growth at different temperatures: the strain grew, at least, within a temperature range between 14 °C and 37 °C.
- Resistance to cycloheximide: the strain presented its growth limit in the presence of 100 ppm of cycloheximide.

### 1.3. Oenological/technological characteristics

Strain GBiot-EL 261 presented rapid, regular fermentation kinetics with a short latency phase. It was capable of performing alcoholic fermentations within a broad range of temperatures, at least between 15 °C and 35 °C, the optimal fermentation temperature being 27 °C.

It had a good flocculating capacity and medium foam formation.

The alcohol yield was high: 16.8 grams of sugar per alcoholic degree produced. It practically exhausted all the reducing sugars present in the grape must, leaving the wines practically dry (between 1 - 5 g/I of residual sugars).

Strain GBiot-EL 261 presented a high resistance to sulfur dioxide, resisting concentrations of up to 75 mg/l of total SO₂. Yeast strain GBiot-EL 261 was seeded in a culture medium containing concentrations of 50, 75 and 100 mg/l of SO₂, and after 48 - 72 h of incubation it was checked for growth.

The tolerance to ethanol was also high, about 16 % vol. The resistance to ethanol was determined by inoculating the autochthonous strain in both a liquid medium and in Petri dishes with ethanol contents of 12 %, 14 % and 16 % v/v.

Strain GBiot-EL 261 presented a neutral *Killer* phenotype, i.e. it did not secrete the *Killer* toxin, but was resistant thereto. The *Killer* phenotype was determined by seeding the yeast to be tested in a plate with a buffered medium inoculated with a lawn of sensitive yeast and, in another plate, with the same medium inoculated with a lawn of *Killer* yeast. Following incubation at 27 °C for 72 h, growth was observed in both plates on both lawns of the control strains, without the appearance of any inhibition haloes.

The production of hydrogen sulfide was low. The method used to determine the formation of H₂S was based on the degree of darkening of the colonies grown in a medium with Biggy agar (*Bismuth-Glucose-Glycine-Yeast*)*,* a modification of the formula developed by Nickerson (1953). This medium contains bismuth sulfite and varies from cream colour (not very productive strains) to dark brown (very productive strains). The degree of darkening is determined by the bismuth sulfide, which is formed by the combination of the bismuth in the medium and the H₂S released by the yeasts.

The production of glycerol was high, with a mean of 11 g/l of glycerol being obtained. The glycerol produced during the alcoholic fermentation was determined using a modification of the enzymatic method developed by Kreutz (1962).

The production of volatile acidity was also low (<0.54 g/I of acetic acid). Garcia-Tena's method was used (Garcia Barceló, 1976).

### 1.4. Genetic characterisation

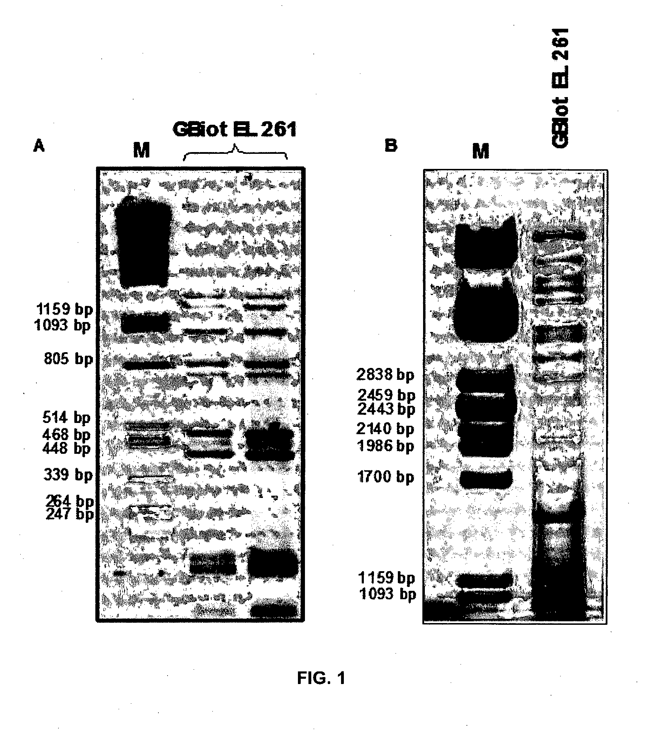

The genetic characterisation of strain GBiot-EL 261 was obtained by means of two complementary molecular techniques: PCR (polymerase chain reaction) amplification of the inter-delta sequences that flank the Ty1 retrotransposons and analysis of the polymorphisms of the restriction fragments of mitochondrial DNA. Figure 1 shows the genetic profiles of strain GBiot-EL 261 obtained with each of the techniques.

### 1.5. Production of biomass

The biomass was obtained by means of a fed-batch culture, in a fermenter with a total capacity of 40 litres (Biostat B), with constant control of the pH, temperature and dissolved oxygen, and under stirring. The feeding was performed using a feeding medium with a total concentration of sugars of 250 g/I (sucrose, glucose, fructose), supplemented with a source of nitrogen, magnesium, vitamins (biotin, inositol and calcium pantothenate) and mineral salts (iron sulfate, zinc sulfate and copper sulfate).

The fermenter was inoculated with a volume of inoculum, ensuring a cell count of 10⁶ CFU/ml in the initial fermentation volume.

As regards the fermentation conditions, they were as follows:
- pH: the pH of the culture was maintained at 4.5 throughout the entire process by the addition of 25 % NaOH (p·v⁻¹) and 75 % H₃PO₄ (p·v⁻¹).
- Temperature: 30 °C.
- Stirring: 700 rpm.

The process for obtaining the biomass lasted about 40 hours. The viability per ml obtained in the fermented broth reached the order of CFU/ml.

From this broth, the following yeast formats were obtained:
- Liquid format: broth fermented without any type of treatment, with a viable cell count of the order of 10⁹ CFU/ml.
- Paste format: it was obtained by filtering the fermented broth using a filter press. This treatment made it possible to obtain a cream with a viable cell count of the order of 10¹⁰ CFU/g.
- Active dry format: this format was obtained from the yeast in paste form by means of a dehydration treatment involving fluid-bed drying. The product must have a moisture content of less than 8 %. The drying at 37 °C for 60 minutes provided a dry yeast product with a viable yeast count greater than 5 x 10¹⁰ CFU/g. When applied, the product must be previously rehydrated.
- Instant dry format: it is obtained from the yeast in paste form by means of a dehydration treatment by fluid-bed drying. The product must have a moisture content of less than 5 % and a viable yeast count ≥ 10¹⁰ CFU/g.

### EXAMPLE 2. OBTAINMENT OF STRAIN GBiot-EL 261

### 2.1. Isolation of yeasts

In order to isolate autochthonous yeasts, healthy, mature grapes of the Tempranillo variety were collected in several vineyards in the viticultural region of the Autonomous Community of La Rioja. Samples were taken from young vineyards, old vineyards and autochthonous vineyards.
- Young vine: vine less than 25 years of age.
- Old vine: vine with an age ranging between 25 - 50 years.
- Autochthonous vine: vine older than 50 years.

The grapes were transferred to the laboratory and stored in a fresh, aerated place until the processing thereof. At this time, they were pressed and fermented in sterile containers. Said containers were introduced into an orbital incubator at a controlled temperature of 27 °C. Throughout the fermentation, there was a follow-up of the °Brix and, once these were stabilised, samples from these containers were taken in order to isolate the yeasts. The samples obtained to isolate the yeasts were seeded, following the corresponding decimal dilutions, in Petri dishes containing malt extract agar and allowed to incubate for three days at 27 °C. Three different colonies the appearance whereof agreed with that of *Saccharomyces cerevisiae* were taken from each plate, containing 30 - 300 colonies. The total number of yeast strains isolated from the grapes which had the appearance of *S. cerevisiae* was 235.

### 2.2. Selection of yeasts

Once all the yeasts were characterised, those which met the following technological requirements were selected:
- Growth in lysine-agar: negative.
- Rapid Tests: *S. cerevisiae.*
- *Killer* phenotype: neutral or *Killer.*
- Formation of hydrogen sulfide: medium or low.
- Resistance to sulfur dioxide: positive growth in plates with at least 50 mg/l SO₂/l.
- Resistance to ethanol: positive growth in plates with at least 12 % of ethanol.

Of all the yeast strains isolated, 146 of the strains were identified as *S. cerevisiae* by means of the Rapid test and PCR amplification of the inter-delta fragments.

The total number of yeasts selected using these criteria was 32. These yeasts were subjected to a first micro-vinification process, in a 50 ml volume, and tested at three temperatures: 15 °C, 27 °C and 35 °C. The micro-vinifications involved inoculating the characterised autochthonous yeasts in a sterile grape must and subsequent laboratory-scale vinification under controlled conditions. Inoculation with the pre-selected autochthonous yeasts was performed in must from red grapes of the Tempranillo variety from the Autonomous Community of La Rioja. Of the 32 yeasts assayed, the three yeasts with the best kinetics at the three temperatures assayed were selected. Subsequently, these three yeasts were subjected to a new vinification process at 27 °C, in a 200 ml volume, with sterile must of the Tempranillo variety, in order to evaluate their organoleptic contribution.

The three fermentations had similar kinetics, and the analytical parameters were similar; however, the wine obtained by the fermentation activity of the strain identified as GBiot-EL 261 was the best valued; for this reason, said strain was selected as the autochthonous yeast for the elaboration of red wine from grapes of the Tempranillo variety from the Autonomous Community of La Rioja.

### EXAMPLE 3. VALIDATION OF THE AUTOCHTHONOUS YEAST GBiot-EL 261 3.1. Laboratory micro-vinifications

Several micro-vinifications were performed with the autochthonous yeast GBiot-EL 261, in liquid format, using different musts from Tempranillo grapes from the Autonomous Community of La Rioja. Parallel to this, micro-vinifications were performed with a commercial yeast, also of autochthonous origin, which was used as a reference.

The vinification processes were performed in flasks containing 200 ml of sterile red grape must of the Tempranillo variety from the viticultural area of La Rioja.

### Micro-vinification I

In this first micro-vinification, the substrate used was a sterile must from Tempranillo grapes from the viticultural area of Upper Rioja, with an initial sugar content of 23.8 °Brix, 342 mg/l of yeast assimilable nitrogen (YAN) and pH 3.38. The fermentations were performed at 27 °C.

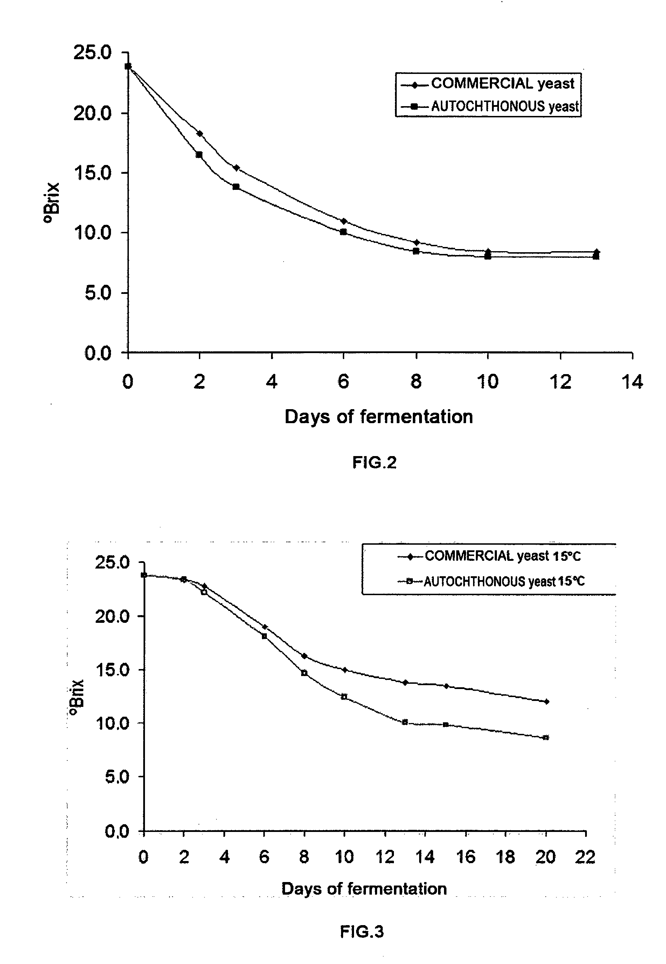

As may be observed in Fig. 2, both fermentations took place without stucks and were completed in 13 days.

The analytical results of the wines obtained from the inoculated grape must were those shown in Table 1.

**Table 1. Analytical parameters of the wines obtained in micro-vinification I.**

| **Analytical parameters** | **Must** | **Wine COMMERCIAL yeast** | **Wine AUTOCHTHONOUS yeast** |
|---|---|---|---|
| pH | 3.38 | 3.43 | 3.35 |
| °Brix | 23.8 | 8.20 | 8.10 |
| Glucose (g/l) | 107.51 | 0.00 | 0.00 |
| Fructose (g/l) | 108.07 | 2.02 | 0.88 |
| Ethanol % (v/v) | 0 | 15.61 | 15.26 |
| Glycerol (g/l) | 0.01 | 8.50 | 10.42 |
| Malic acid (g/l) | 2.88 | 2.42 | 2.72 |
| Total acidity (g/l tartaric acid) | 3.2 | 4.66 | 4.42 |
| Volatile acidity (g/l acetic acid) | 0.24 | 0.48 | 0.43 |

The autochthonous yeast GBiot-EL 261 made it possible to obtain wine with a lower fructose content and a higher glycerol content as compared to the wine elaborated with the commercial yeast of autochthonous origin.

### Aroma analysis of micro-vinification I

In order to evaluate the organoleptic contribution of the autochthonous yeast GBiot-EL 261 to the wine aroma, the aromatic compounds of the wines obtained in micro-vinification I were analysed by means of GC chromatography-MS.

Table 2 shows the data relative to the compounds wherein significant differences were observed. The results correspond to the means of the two micro-vinifications performed with each of the yeasts.

**Table 2. Aromatic composition of the wines obtained in micro-vinification I, inoculated with the autochthonous yeast GBiot-EL 261 and the commercial yeast.**

| **Volatile compounds (mg/l)** | **Wine AUTOCHTHONOUS yeast** | **Wine COMMERCIAL yeast** |
|---|---|---|
| Ethyl lactate | 3 | 2 |
| Diethyl succinate | 0.27 | 0.25 |
| Ethyl hexanoate | 0.136 | 0.097 |
| Ethyl isovalerate | 0.0012 | 0.0009 |
| Ethyl butyrate | 0.052 | 0.04 |
| Ethanal | 47.5 | 62.5 |
| Ethyl acetate | 18.5 | 21 |

The wines inoculated with the autochthonous yeast GBiot-EL 261 had a higher concentration of ethyl esters and a lower concentration of ethanal and ethyl acetate.

Ethyl succinate and ethyl lactate are two ethyl esters related to dairy and coffee aromas. In particular, ethyl lactate contributes to mitigate the bitter and acid edges in the flavour and, according to some authors, contributes to enrich the volume and the roundness in the mouth.

Ethyl hexanoate is related to fruity aroma descriptors, specifically pineapple and apple aromas, whereas ethyl isovalerate and ethyl butyrate contribute aromas from forest fruits, both red (strawberries, raspberries, etc.) and black (blackberries, cranberries, etc.), characteristic of red Rioja wines.

Most of the ethanal or acetaldehyde is produced by the yeasts at the beginning of the alcoholic fermentation. When in excess, it is responsible for the typical oxidised wine odour, with Sherry or excessively ripe apple aromas that are not desirable in any table wine.

Acetic acid may react with ethanol, to produce ethyl acetate, a compound that gives the wine a glue, hairspray or nail polish odour. Since both compounds result from the oxidative degradation of wine, the acetic acid odour and the glue odour often appear simultaneously in wine; for this reason, a lower final concentration in the wine is desirable. In fact, at low concentrations ( < 50 mg/l), it may be positive and contribute complexity to the aroma.

In sum, the results obtained in the chromatographic analysis of the wines obtained in micro-vinification I show that the autochthonous yeast GBiot EL-261 produces a greater quantity of ethyl esters, the sensory descriptors whereof correspond to fruity aromas, particularly of forest fruits. This characteristic reinforces their autochthonous character and their respect for the varietal character and the typicality of Rioja wines.

Moreover, the wines obtained in this micro-vinification were tasted by several oenologists from cellars in the Autonomous Community of La Rioja. In the organoleptic evaluation, based on the appreciation of the wine quality in the nose and mouth, both wines were correct from an organoleptic standpoint, and no defects were noted. The wine inoculated with the autochthonous yeast GBiot-261 was the best valued by the panel of tasters. In the wine obtained with the yeast GBiot-EL 261, the sensory evaluation stressed the presence of red and black fruits (strawberries, blackberries, blueberries) in the nose, as well as a very good acidity and volume in the mouth, which results in a very balanced wine that is persistent in the mouth. This sensory evaluation agrees with the analytical results obtained by means of GC-MS.

### Micro-vinification II

In order to evaluate the fermentation activity of the new yeast GBiot-EL 261 under stressful fermentation conditions, and thus evaluate its fermentation robustness, a low-temperature fermentation was performed, specifically at 15 °C. The grape must used was the same as in the previous micro-vinification and it was also sterilised, as in the previous case.

As may be observed in Fig. 3, the autochthonous yeast showed a better adaptation to low temperature and made it possible to initiate the fermentation process earlier than the commercial yeast. As regards the analytical results of the wines obtained in this micro-vinification, they are shown in Table 3.

**Table 3. Analytical parameters of the wines obtained in micro-vinification II.**

| **Analytical parameters** | **Must** | **Wine COMMERCIAL yeast** | **Wine AUTOCHTHONOUS yeast** |
|---|---|---|---|
| °Brix | 23.8 | 8.80 | 8.40 |
| Glucose (g/l) | 107.51 | 0.98 | 4.53 |
| Fructose (g/l) | 108.07 | 50.04 | 20.95 |
| Ethanol % (v/v) | 0 | 15.60 | 14.98 |
| Glycerol (g/l) | 0.01 | 9.85 | 10.68 |
| Malic acid (g/l) | 2.88 | 2.79 | 2.01 |
| Total acidity (g/l tartaric acid) | 3.2 | 3.56 | 3.71 |
| Volatile acidity (g/l acetic acid) | 0.24 | 0.65 | 0.51 |

As may be observed, the autochthonous yeast GBiot-EL 261 provided wine with a lower sugar content and a higher glycerol content, as was the case in the micro-vinifications performed at 27 °C. The volatile acidity was also lower in the wine elaborated with the autochthonous yeast GBiot-EL 261.

### Micro-vinification III

In order to continue to evaluate the kinetic robustness of the autochthonous yeast, micro-vinifications were performed with sterile grape musts at 35 °C. The must used was the same as in the previous micro-vinifications.

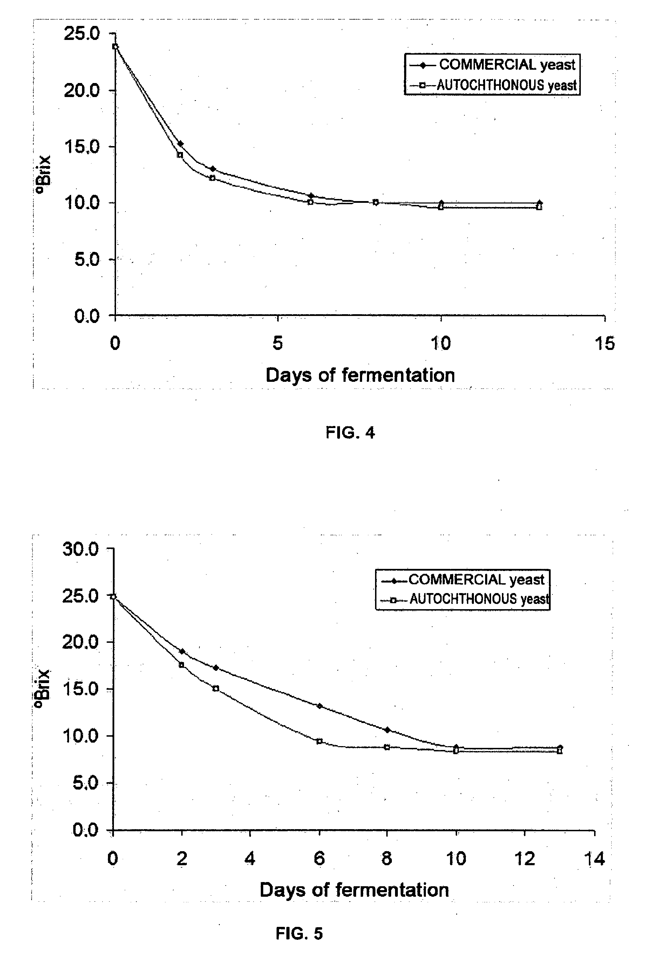

As shown in Figure 4, both yeasts performed the fermentation in the same period of time. As in the previous micro-vinification, the adaptation of the autochthonous yeast GBiot-EL 261 to stressful conditions was faster than that of the commercial yeast.

Table 4 shows the results of the analyses performed on the wines obtained under these conditions.

**Table 4. Analytical parameters of the wines obtained in micro-vinification III.**

| **Analytical parameters** | **Must** | **Wine COMMERCIAL yeast** | **Wine AUTOCHTHONOUS yeast** |
|---|---|---|---|
| °Brix | 23.8 | 10.00 | 9.60 |
| Glucose (g/l) | 107.51 | 1.80 | 1.07 |
| Fructose (g/l) | 108.07 | 13.92 | 13.75 |
| Ethanol % (v/v) | 0 | 9.96 | 9.27 |
| Glycerol (g/l) | 0.01 | 10.20 | 12.46 |
| Malic acid (g/l) | 2.88 | 2.68 | 2.98 |
| Total acidity (g/l tartaric acid) | 3.2 | 2.35 | 3.78 |
| Volatile acidity (g/l acetic acid) | 0.24 | 0.63 | 0.43 |

In this micro-vinification, it was observed that the wine elaborated with the autochthonous yeast GBiot-EL 261 presented a higher glycerol content and a lower volatile acidity than that obtained with the commercial yeast of autochthonous origin.

### Micro-vinification IV

In this micro-vinification, the fermentation capacity of both yeasts was evaluated in grape musts with a high initial sugar content. Sugar was added to the same sterile must used in the previous micro-vinifications until 25 °Brix was reached. The temperature of the fermentations was 27 °C.

Figure 5 shows that the fermentation kinetics with high concentrations of sugar was faster and more regular with the autochthonous yeast. The beginning of fermentation was faster and the residual sugars at the end of fermentation were lower, particularly fructose. Once again it was observed that the autochthonous yeast GBiot-EL 261 adapts better to stressful conditions.

Table 5 shows the analytical results of the wines obtained from grape must with a high sugar content.

**Table 5. Analytical parameters of the wines obtained in micro-vinification IV.**

| **Analytical parameters** | **Must** | **Wine COMMERCIAL yeast** | **Wine AUTOCHTHONOUS yeast** |
|---|---|---|---|
| °Brix | 25 | 8.80 | 8.40 |
| Glucose (g/l) | 118.85 | 0.80 | 0.60 |
| Fructose (g/l) | 116.18 | 2.06 | 0.79 |
| Ethanol % (v/v) | 0 | 11.21 | 11.19 |
| Glycerol (g/l) | 0.01 | 9.18 | 11.14 |
| Malic acid (g/l) | 2.88 | 2.67 | 2.11 |
| Total acidity (g/l tartaric acid) | 3.2 | 2.73 | 1.61 |
| Volatile acidity (g/l acetic acid) | 0.24 | 0.65 | 0.48 |

As in the previous micro-vinifications, it was observed that the wine elaborated with the autochthonous yeast GBiot-EL 261 contained a higher concentration of glycerol and a lower volatile acidity than that obtained with the commercial yeast of autochthonous origin. In addition, a more fructophilic character of the yeast GBiot-EL 261 was once again observed, as shown in the fructose value obtained for the wine elaborated with the autochthonous yeast GBiot-EL 261.

### 3.2. Consumption of sugars

In wine must, glucose and fructose are the primary fermentable sugars and are generally found in equal quantities in the grape must. *S. cerevisiae* is a glucophilic yeast, which shows a greater preference for glucose than fructose. During alcoholic fermentation, the glucose consumption ratio is much greater than the fructose consumption ratio; for this reason, at the end of the process, the concentration of residual fructose is higher. Some authors state that the differences in the glucose and fructose utilisation ratios of *S. cerevisiae* strains during alcoholic fermentation may be attributed to differences in the transport efficiency of the two sugars. Others suggest that the preference of *S. cerevisiae* for glucose over fructose is dependent on the strain and that the difference in consumption between glucose and fructose is not a fixed parameter, but one dependent on the genetic history of the yeast and the external conditions.

A good fructose consumption capacity is of great interest, since it guarantees having less fermentation problems, including the fermentation stucks caused by high initial sugar levels.

In order to check whether there were differences in the consumption of fructose between the autochthonous yeast strain isolated from grapes from La Rioja vineyards and the reference commercial yeast, a series of laboratory micro-vinification processes were performed, inoculating each of the yeasts in fermentation flasks containing MS300 synthetic must, which simulates the composition of grape must. The initial conditions of the must were modified, by changing the initial concentration of sugars: (i) Standard conditions: 100 g/I glucose + 100 g/I fructose; (ii) Excess fructose: 50 g/I glucose + 150 g/I fructose; (iii) Only fructose: 200 g/I fructose; (iv) Only glucose: 200 g/I glucose. In all cases, the initial concentration of yeast assimilable nitrogen (YAN) was 300 mg/l and the pH was 3.47. The micro-vinifications were performed in triplicate, inoculating 2 x 10⁶ CFU/ml in a total volume of 30 ml, at 27 °C, under stirring.

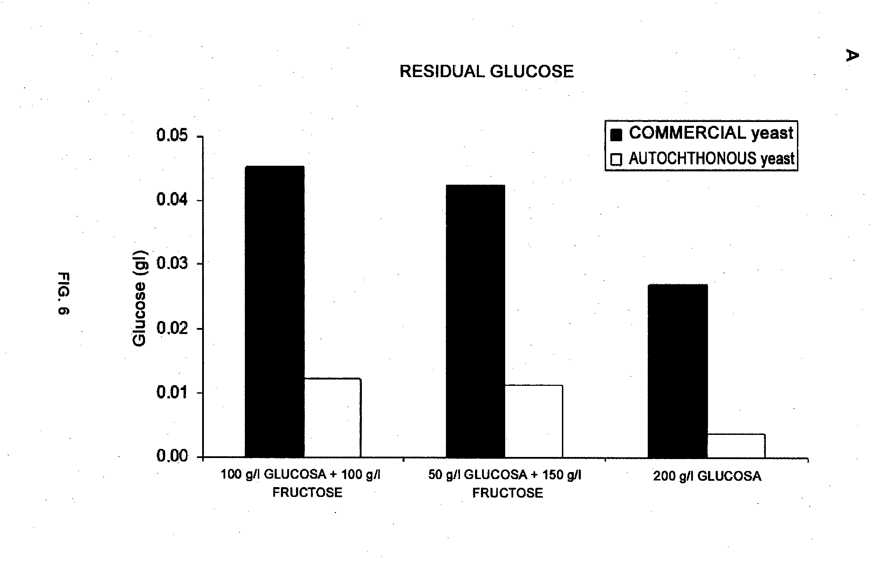

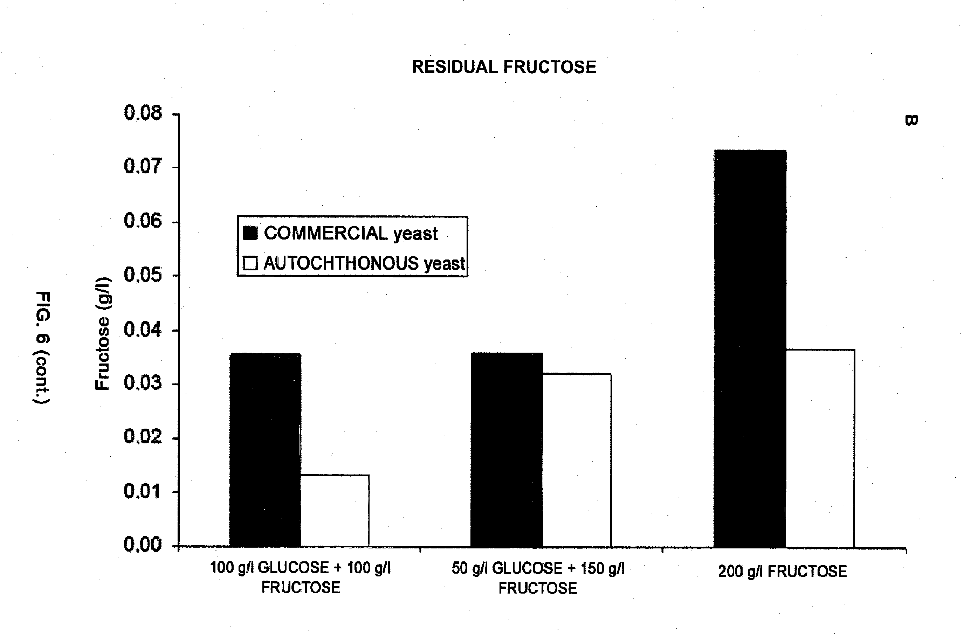

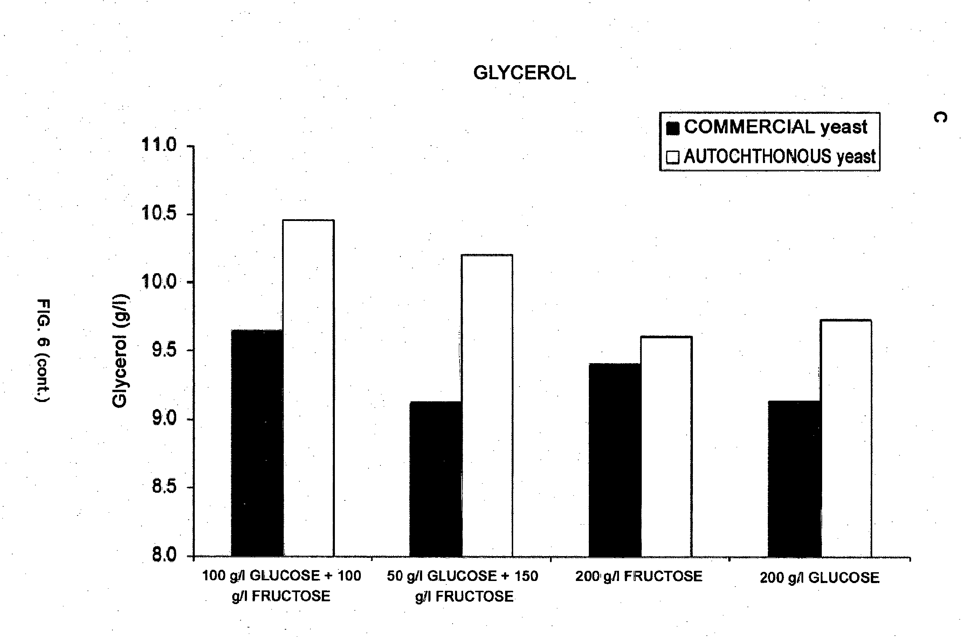

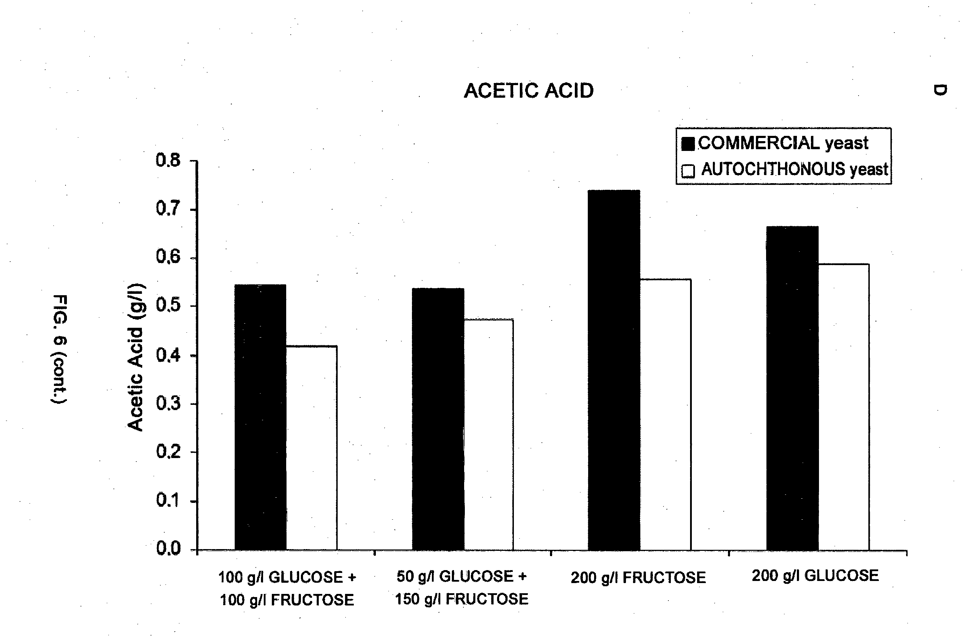

After 8 days of fermentation had elapsed, and once all the micro-vinifications were completed, samples were taken and the residual sugars, as well as the glycerol and the acetic acid, of the wines obtained were quantified by means of HPLC (Fig. 6).

The results obtained in the micro-vinifications performed with different sugar contents and concentrations allowed us to conclude the following:
- The concentrations of both residual glucose and residual fructose were lower in all the grape musts inoculated with the autochthonous yeast GBiot-EL 261 (Figs. 6A and 6B), the consumption of fructose being of particular interest.
- The production of glycerol by the autochthonous yeast GBiot-EL 261 was greater under the different conditions studied, in all cases exceeding 9.6 g/I (Fig. 6C).
- Finally, the volatile acidity (g/I of acetic acid) in the wines fermented with the autochthonous yeast GBiot-EL 261 was lower (Fig. 6D).

### 3.3. Mannoprotein content in the cell wall

Mannoproteins are majority components (25 % - 50 %) of the cell wall of *Saccharomyces cerevisiae* yeasts and, together with other polysaccharides, form a part of the structure thereof. They are released during the active phase of growth of cells and following cell death, during the so-called autolytic phase.

These compounds are of great oenological interest for several reasons:
- They improve the protein and tartaric stability of wines.
- They improve the stability of the colouring matter.
- They reduce the astringency of red wines.
- They improve the aromatic persistence.
- They improve the foam characteristics.
- They increase the untuousity, the body, the sweetness and the roundness of wine.

The release of mannoproteins during alcoholic fermentation is dependent on several factors, one of the main ones being the yeast strain. Certain *Saccharomyces cerevisiae* yeast strains exhibit a marked production of these macromolecules during fermentation, and this factor is one of the new selection criteria for yeasts designed for alcoholic fermentation.

In order to evaluate the release of mannoproteins by the yeasts, the resistance to the K9 toxin was studied. The yeast *Williopsis saturnus* var. *mrakii* produces the K9 toxin, which inhibits cell wall synthesis and the activity of ß-1,3-glucan synthase, an enzyme that synthesises ß-1,3-glucans, structural macromolecules that, jointly with mannoproteins, compose the cell wall of yeasts. Yeasts with a higher concentration of mannoproteins reduce the wall permeability and, thus, limit the access of the K9 toxin to the ß-1,3-glucan layer, making the cells more resistant.

In order to produce the toxin, a cell culture of the yeast *Williopsis saturnus* var. *mrakii* (CECT 11031) in YPD medium was made, incubating it at 19 °C and 150 rpm for 4 days. Once the growth was completed, the supernatant containing the toxin was centrifuged and filtered (0.22 µm). On the other hand, cultures were made of the autochthonous yeast GBiot-EL 261 and the commercial yeast in liquid YPD medium for 24 hours. Inocula of both yeasts were prepared in order to reach an initial population of 10³ CFU/ml, in 4 tubes containing different concentrations of the K9 toxin: 0 %, 25 %, 50 % and 100 % (v/v), and allowed to incubate for 5 hours at 27 °C. After this time had elapsed, 5 µl aliquots were taken from each of the tubes and seeded in YPD plates at 27 °C. Readings of the results of the growth of the yeasts was performed at 24 and 48 h.

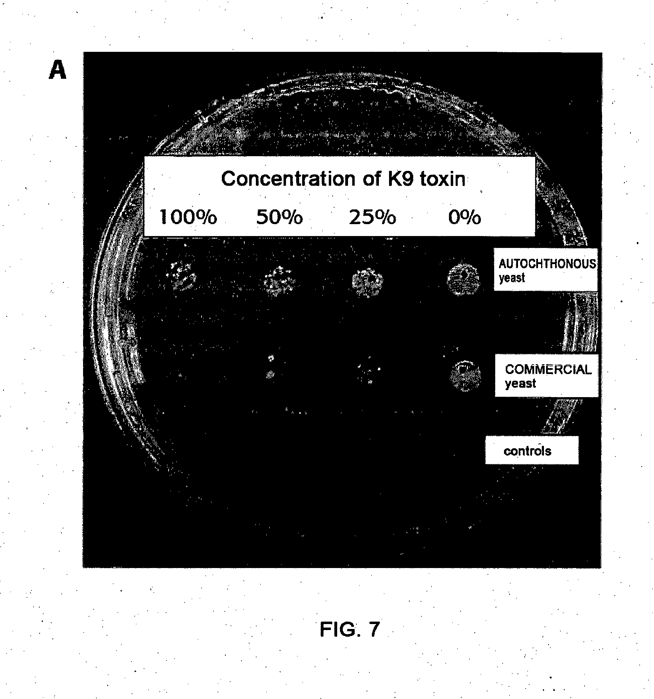

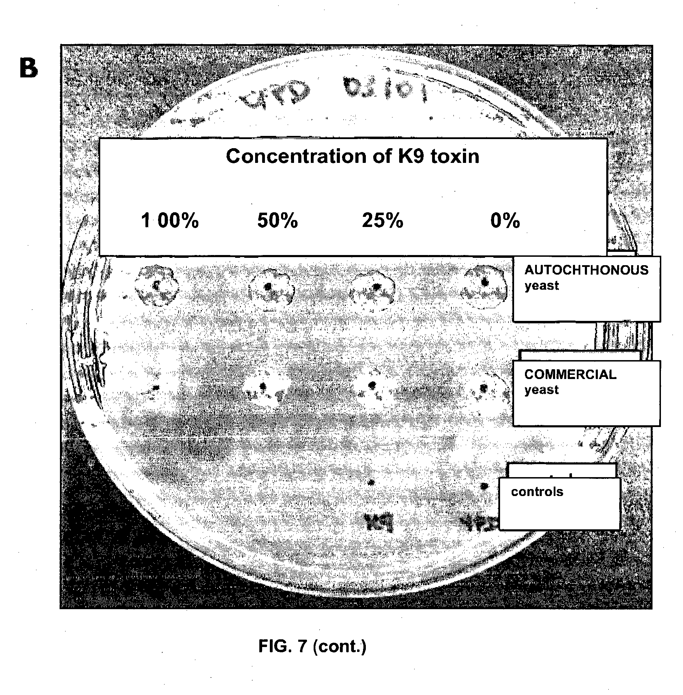

As may be observed in Figure 7, the growth of the autochthonous yeast GBiot-EL 261 at 24 (Fig. 7A) and 48 hours (Fig. 7B) was greater, being particularly evident at 24 hours. It was also observed that, at 48 hours and a 100 % concentration of the K9 toxin, the autochthonous yeast GBiot EL-261 presented a greater resistance, as may be seen in the growth, which is much greater.

Therefore, it may be concluded that the autochthonous yeast GBiot EL-261 has a higher mannoprotein content in the structure of its cell wall, and that its contribution to the stabilisation and the organoleptic properties that improve wine quality is greater than that of commercial yeast.

### 3.4. Conclusions

The results obtained in the validation assays performed with the autochthonous yeast strain GBiot-EL 261 and the commercial yeast of autochthonous origin made it possible to draw the following general conclusions:
- The autochthonous yeast GBiot-EL 261 presented a high, regular fermentation capacity at different fermentation temperatures and with different sugar contents in the grape must.
- In grape musts with both 23 °Brix and 25 °Brix, the autochthonous yeast GBiot-EL 261 initiated fermentation more vigorously. The commercial yeast had a slower beginning of fermentation. In any case, both the commercial and the autochthonous yeasts ended the fermentation within a similar period of time.
- The wines elaborated from sterile Tempranillo must and with the autochthonous yeast GBiot-EL 261 presented a higher glycerol content and a lower fructose content, as well as a lower volatile acidity, as compared to the wines elaborated with the commercial yeast of autochthonous origin.
- The sensory analysis performed made it possible to highlight the autochthonous character of the yeast, which respected and maintained the typicality of wines elaborated with the Tempranillo grape variety.
- The autochthonous yeast GBiot EL-261 presents a higher mannoprotein content in the structure of its cell wall; for this reason, we may expect to obtain a more stable wine, with better organoleptic properties than that which may be obtained with the commercial yeast.

As a general conclusion, it may be stated that, due to its kinetic and oenological characteristics and its organoleptic contribution, the autochthonous yeast GBiot-EL 261 has a high technological quality and is suitable for the elaboration of wines of the Tempranillo variety from the Autonomous Community of La Rioja.

### EXAMPLE 4. INDUSTRIAL FERMENTATIONS

The autochthonous yeast selected, analysed in the laboratory-scale micro-vinification assays, was tested during the 2011 grape harvest in two Upper Rioja cellars. In addition to this yeast, the same commercial yeast was used which was assayed in the micro-vinifications as a control. The two yeasts were assayed in reservoirs containing Tempranillo grape must.

The autochthonous yeast was supplied to the cellars in fresh in paste format. The commercial yeast was assayed in the form of active dry yeast, which is the commercially available format.

Below we show the results for each of cellars A and B (Tables 6 and 7, respectively).

**Table 6. Analytical parameters of the wines obtained in CELLAR A.**

| | **COMMERCIAL yeast** | | **AUTOCHTHONOUS yeast** | |
|---|---|---|---|---|
| **Analytical parameters** | **Must** | **Wine** | **Must** | **Wine** |
| pH | 3.3 | 3.20 | 3.30 | 3.30 |
| °Brix | 22.6 | 8.60 | 22.0 | 8.80 |
| Glucose (g/l) | 101.51 | 0.00 | 99.04 | 0.00 |
| Fructose (g/l) | 121.01 | 0.00 | 116.07 | 0.00 |
| Ethanol % (v/v) | 0 | 13.98 | 0.00 | 14.00 |
| Glycerol (g/l) | 1.1 | 9.66 | 0.00 | 10.94 |
| Malic acid (g/l) | 1.3 | 2.20 | 1.50 | 2.60 |
| Total acidity (g/l tartaric acid) | 6.68 | 5.88 | 5.86 | 5.84 |
| Volatile acidity (g/l acetic acid) | < 0.10 | 0.32 | < 0.10 | 0.31 |

**Table 7. Analytical parameters of the wines obtained in CELLAR B.**

| | **COMMERCIAL yeast** | | **AUTOCHTHONOUS yeast (Reservoir 1)** | | **AUTOCHTHONOUS yeast (Reservoir 2)** | |
|---|---|---|---|---|---|---|
| **Analytical parameters** | **Must** | **Wine** | **Must** | **Wine** | **Must** | **Wine** |
| pH | 3.2 | 3.5 | 3.4 | 3.1 | 3.4 | 3.4 |
| °Brix | 21.2 | 8.00 | 23.70 | 8.80 | 21.50 | 8.00 |
| Glucose (g/l) | 93.48 | 0.00 | 114.21 | 0.00 | 100.84 | 0.00 |
| Fructose (g/l) | 102.31 | 2.02 | 121.22 | 0.88 | 108.77 | 0.88 |
| Ethanol % (v/v) | 0 | 11.76 | 0.00 | 13.75 | 0.00 | 12.70 |
| Glycerol (g/l) | 0 | 9.60 | 0.00 | 10.42 | 0.00 | 10.14 |
| Malic acid (g/l) | 1.7 | 2.50 | 2.00 | 2.70 | 2.60 | >3 |
| Total acidity (g/l tartaric acid) | 5.26 | >10 | 5.74 | 4.66 | 5.58 | 3.68 |
| Volatile acidity (g/l acetic acid) | < 0.10 | 0.21 | < 0.10 | 0.14 | < 0.10 | < 0.10 |

At the cellar level, the same trend as in the laboratory-level micro-vinifications was observed, such that wines with a higher glycerol content and a lower volatile acidity were obtained. In the wines from cellar B, a lower fructose content was also observed, which once again corroborated the greater fructophilic character of the autochthonous yeast GBiot-EL 261.

In addition to the physico-chemical characterisation of the wines obtained, implantation of the yeast was checked against the indigenous population present in the grape must. The implantation study was performed by studying the genetic profiles of strain GBiot-EL 261 obtained by electrophoretic separation in agarose gels using amplification of the inter-delta sequences and determination of the *Killer* phenotype. The implantation results are shown in Tables 8 and 9.

**Table 8. Implantation results in Cellar A.**

| **Inoculated yeast** | **Agreement inter-δ profile + *Killer* phenotype** | **Degree of implantation** |
|---|---|---|
| COMMERCIAL yeast | 100% | IMPLANTED |
| AUTOCHTHONOUS yeast | 100 % | IMPLANTED |

Both the autochthonous yeast GBiot EL-261 and the commercial yeast were implanted in the reservoirs inoculated therewith in Cellar A. Both the molecular profile and the *Killer* phenotype of 100 % of the isolates obtained in the wine samples fermented with the yeasts agreed with the strain inoculated in the reservoir.

**Table 9. Implantation results in Cellar B.**

| **Inoculated yeast** | **Agreement inter-δ profile + *Killer* phenotype** | **Degree of implantation** |
|---|---|---|
| COMMERCIAL yeast | 100 % | IMPLANTED |
| AUTOCHTHONOUS yeast (Reservoir 1) | 100 % | IMPLANTED |
| AUTOCHTHONOUS yeast (Reservoir 2) | 100 % | IMPLANTED |

As in the case of Cellar A, both the autochthonous yeast GBiot EL-261 and the commercial yeast were implanted in the reservoirs inoculated therewith in Cellar B. The molecular profile and the *Killer* phenotype of 100 % of the isolates agreed with the yeast inoculated.

### Conclusions

The results obtained with the industrial fermentations performed with the autochthonous yeast in fresh in paste format and the commercial yeast made it possible to draw the following general conclusions:
- The autochthonous yeast GBiot-EL 261 presented good implantation qualities.
- The autochthonous yeast GBiot-EL 261 presented a high fermentation capacity and resulted in wines with an adequate alcoholic degree for Rioja wines. Moreover, a good yield in the production of ethanol was achieved.
- The autochthonous yeast GBiot-EL 261 produced a greater quantity of glycerol and a lower volatile acidity than the commercial yeast.
- The autochthonous yeast GBiot-EL 261 presented a greater fructophilic character.

## Claims

1. Strain of the species *Saccharomyces cerevisiae* deposited in the Spanish Type Culture Collection with deposit number CECT 13065.

2. Strain according to claim 1, **characterised in that** it is found in liquid format, fresh in paste, active dry or instant dry.

3. Use of the strain according to any of claims 1 or 2, for the elaboration of alcoholic beverages obtained by alcoholic fermentation.

4. Use according to claim 3, wherein the alcoholic beverage is selected from the list consisting of: wine, beer, cava and cider.

5. Use according to claim 4, wherein the alcoholic beverage is wine elaborated from grape musts.

6. Use according to claim 5, wherein the grape is of the Tempranillo variety.

7. Use of the strain according to any of claims 1 or 2, for the production of biomass.

## Patentansprüche

1. Stamm der Spezies *Saccharomyces cerevisiae,* der in der spanischen Typenkultursammlung mit der Hinterlegungsnummer CECT 13065 hinterlegt ist.

2. Stamm nach Anspruch 1, **dadurch gekennzeichnet, dass** dieser in flüssiger Form, frisch in einer Paste, aktiv trocken oder sofort trocken angetroffen wird.

3. Die Verwendung des Stamms nach einem der Ansprüche 1 oder 2 zur Erzeugung alkoholischer Getränke, die durch alkoholische Gärung hergestellt werden.

4. Die Verwendung des Stamms nach Anspruch 3, wobei das alkoholische Getränk aus einer Liste ausgewählt wird, die aus Folgendem besteht: Wein, Bier, Cava und Cider.

5. Die Verwendung des Stamms nach Anspruch 4, wobei es sich bei dem alkoholischen Getränk um Wein handelt, der aus Traubenmosten hergestellt wird.

6. Die Verwendung des Stamms nach Anspruch 5, wobei es sich bei den Trauben um die Rebsorte Tempranillo handelt.

7. Die Verwendung des Stamms nach einem der Ansprüche 1 oder 2 zur Herstellung von Biomasse.

## Revendications

1. Souche de l'espèce *Saccharomyces cerevisiae* déposée dans la CECT (Collection espagnole de cultures type) sous le numéro de dépôt CECT 13065.

2. Souche selon la revendication 1, **caractérisée en ce qu'**on la trouve sous forme liquide, fraîche en pâte, sèche active ou sèche instantanée.

3. Utilisation de la souche selon n'importe laquelle des revendications 1 ou 2, pour l'élaboration de boissons alcoolisées obtenues par fermentation alcoolique.

4. Utilisation selon la revendication 3, dans laquelle la boisson alcoolisée est sélectionnée dans la liste composée de : vin, bière, cava et cidre.

5. Utilisation selon la revendication 4, dans laquelle la boisson alcoolisée est du vin élaboré à partir de moûts de raisin.

6. Utilisation selon la revendication 5, dans laquelle la variété de raisin est le Tempranillo.

7. Utilisation de la souche selon n'importe laquelle des revendications 1 ou 2, pour la production de biomasse.
